Europäisches Patentamt

European Patent Office

Office européen des brevets

(11) Publication number: **0 154 505 B1**

## EUROPEAN PATENT SPECIFICATION

(45) Date of publication of patent specification: **30.09.92**   (51) Int. Cl.⁵: **C12Q 1/68**

(21) Application number: **85301325.8**

(22) Date of filing: **27.02.85**

(54) **Diagnosis of gene abnormalities by restriction mapping using a sandwich hybridization format.**

(30) Priority: **28.02.84 US 584415**

(43) Date of publication of application:
**11.09.85 Bulletin  85/37**

(45) Publication of the grant of the patent:
**30.09.92 Bulletin  92/40**

(84) Designated Contracting States:
**DE FR GB**

(56) References cited:
**EP-A- 0 070 685**      **EP-A- 0 101 228**
**EP-A- 0 130 515**      **EP-A- 0 139 489**
**WO-A-84/03520**        **US-A- 4 329 152**

(73) Proprietor: **ORTHO DIAGNOSTIC SYSTEMS INC.**
**One Johnson & Johnson Plaza**
**New Brunswick New Jersey 08933-7003(US)**

(72) Inventor: **Monahan, John E.**
**4105 Stearns Hill Road**
**Waltham, MA 02154(US)**
Inventor: **Ip, Stephen H. C.**
**3 Heather Lane**
**Mansfield, MA 02048(US)**
Inventor: **Rittershaus, Charles**
**65 Garden Street**
**Malden, MA 02148(US)**

(74) Representative: **Jones, Alan John et al**
**CARPMAELS & RANSFORD 43 Bloomsbury Square**
**London, WC1A 2RA(GB)**

Rank Xerox (UK) Business Services

EP 0 154 505 B1

## Description

### Field of the Invention

This invention relates to assays in general, and in particular to those useful for detecting the presence or absence of a specific genetic sequence in a nucleic acid sample.

### Background of the Invention

It has long been recognized that numerous disease states may be related to the presence of genetic abnormalities in an individual's chromosomes. Such genetic abnormalities often occur due to undesirable changes of nucleotide sequences in the nucleic acid strands carrying the genetic information. Transcription and translation of these erroneous sequences often results in nonfunctional proteins and in some instances, the abnormal sequences may actually prevent or inhibit transcription and/or translation. Consequently, identification of the presence of these abnormal genetic sequences is often diagnostically valuable.

It is an object of the present invention to provide an assay which is easily capable of identifying the presence or absence of specific genetic sequences associated with abnormalities.

Conventional methods for detecting specific sequences in DNA fragments have relied upon separation by gel electrophoresis. See Southern, E.M., "Detection of Specific Sequences Among DNA Fragments Separated By Gel Electrophoresis", J. Mol. Biol. 98:503-517 (1975). Such methods have been employed for studying the structure of DNA and typically require reduction of the DNA into fragments which result from cleavage by restriction endonucleases. The fragments are separated by electrophoresis techniques in agarose or polyacrylamide gels, denatured and immobilized onto a membrane surface, and then hybridized with radioactively labeled single stranded DNA or polynucleotide probe as a means of identifying the thusly separated fragmentary pieces of sample DNA. Such methods are disadvantageously time consuming, labor intensive and are often incapable of identifying fragments of 500 nucleotide pairs or less depending upon the membrane surface or technique employed.

It is an object of the present invention to provide methods that have fewer steps and are accordingly simpler and less time consuming.

U.S. Patent No. 4,358,535 to Falkow et al., describes one class of methods directed to nucleic acid assays involving specific DNA probes in diagnostic microbiology. This method involves denaturization of the DNA and fixation thereof onto a support whereupon a labeled polynucleotide probe, specific for a DNA sequence, is contacted with the nucleic acid under hybridizing conditions. Hybridization of the probe to the single stranded nucleic acid is diagnostic of the presence of the pathogen. Such a method, however, fails to provide or teach how sequences having a limited number of differences distinquishing between an abnormal and normal gene can be detected. Although the Falkow technique could theoretically detect a single mutation, to do so would disadvantageously require the additional steps of culturing the bacteria, harvesting, and purifying the DNA.

EP-A-0130515 discloses a method for determining the presence of a specific sequence present in a double stranded nucleic acid. The method uses a labelled probe and a probe immobilised to a solid support. Detection of the specific sequence depends upon the dual hybridisation of the denatured nucleic acid with both of the probes.

It is an object of the present invention to employ DNA probes but in a far more useful format without reliance on methods requiring many steps and, in particular, to provide methods which can detect the presence of point mutations rather than identifying comparatively lengthy gene sequences.

Other methods of nucleic acid detection in samples have been described employing sandwich hybridization techniques and include for instance that described by Ranki et al., "Sandwich Hybridization As A Convenient Method For The Detection of Nucleic Acids In Crude Samples", Gene 21:77-85 (1983). As with the Falkow et al. methods, the Ranki and related methods require hybridization of a nucleic acid probe with the sequences of interest and accordingly, rely upon the availability of such a probe having homologous sequences. Such methods are, however, generally only useful when large genetic sequences are sought to be identified such as for instance, the presence of cytomegalovirus DNA in clinical urine specimens. See Chou et al., "Rapid Detection and Quantitation of Human Cytomegalvirus in Urine Through DNA Hybridization", New England Journal of Medicine, Vol. 308, 16:921-925 (1983).

Antonarakis et al. described another method of genetic disease diagnosis in an article entitled "Genetic Diseases: Diagnosis By A Restriction Endonuclease Analysis", Journal of Pediatrics, Vol. 100, 6:845-856 (1982). This method involves the digestion of DNA with a restriction endonuclease, size separation by electrophoresis, denaturization, and Southern blot immobilization of the resultant single stranded fragments. Identification of the individual fragments is accomplished by hybridization with isotopically labeled single stranded DNA sequences and the hybridized sample then subjected to autoradiography. Such methods disadvantageously require labeled probes to hybridize with the regions of interest following a general endonuclease digestion and size separation of the motley collection of DNA fragments.

It is an object of the present invention to instead employ a specific restriction endonuclease reactive at the site of interest only in special circumstances and DNA probes capable of hybridization with the remaining portions of the sample DNA thereby facilitating production of the necessary reagents specifically, and the detection method generally.

Recent investigations have revealed detailed information concerning the genetic structures associated with various disease states. Such information has been reported in a number of articles and include for instance, the following: Boehm et al., "Prenatal Diagnosis Using DNA Polymorphisms", New England Journal of Medicine, Vol 308, 18:1054-1058 (1983); Pirastu et al., "Prenatal Diagnosis of $\beta$-Thalassemia", New England Journal of Medicine, 309, 5:284-287 (1983); Conner et al., "Detection of Sickle Cell $\beta$-globin Allele By Hybridization With Synthetic Oligonucleotides", Proc. Natl. Acad. Sci., 80:278-282 (1983); and Orkin et al., "Linkage of $\beta$-Thalassaemia Mutations And $\beta$-globin Gene Polymorphisms With DNA Polymorphisms In Human $\beta$-globin Gene Cluster", Nature, Vol. 296:627-631 (1982), all of which describe various genetic abnormalities associated with blood disorders. Another government funded source for more than 2700 DNA and RNA sequences is the centralized computer library called GenBank.

It is an object of the present invention to capitalize on the information revealed by such investigations, specifically that concerning disease associated genetic sequences.

It is another object of the present invention to rely upon the vast array of restriction endonucleases which have recently become available.

It is yet another object to employ nucleic acid probes which may be immobilized or labelled in a hybridization scheme.

It is still yet another object to combine the aforementioned recently available reagents and knowledge in a hybridization format employing aqueous and solid phases whereby the presence of a single nucleic acid sequence change may be detected.

It is still another object of the present invention whereby the presence of point mutations in nucleic acid sequences may be detected with methods which measure agglutination or changes in agglutination patterns which may be related to the presence or absence of the nucleic acid sequence of interest.

According to the first aspect of the present invention there is provided a method for determining the presence of a specific sequence occurring within a double stranded nucleic acid in an aqueous sample comprising:

a) providing an immobilized nucleic acid probe complementary to a first sequence on either the upstream or the downstream side of said specific sequence;

b) further providing a labelled nucleic acid probe complementary to a second sequence on the other of the upstream or downstream side of said specific sequence;

c) contacting said double stranded nucleic acid in an aqueous sample

either with a restriction endonuclease which will cleave said nucleic acid strand at said specific sequence if present but not at any site between or within said first and second sequences whereby said nucleic acid strand is digested into at least two portions if said specific sequence is present

or with a restriction endonuclease which will not cleave said nucleic acid strand at said specific sequence if said specific sequence is present nor at any other site between said first or second sequence and said specific sequence or within said first and second sequences whereby said first and second sequences remain connected if said specific sequence is present;

d) denaturing said double stranded nucleic acid into single stranded nucleic acid after said contacting step;

e) reacting said single stranded nucleic acid with said immobilized nucleic acid probe and said labelled nucleic acid probe under conditions which permit hybridization to occur;

f) separating said immobilized probe from said aqueous sample; and

g) determining the presence of said specific sequence on the basis of whether or not said labelled probe is associated with said immobilized probe or with said aqueous sample following said separation step;

wherein said immobilized nucleic acid probe is immobilized by attachment to microbeads, latex particles, a plastic surface, a glass surface or a porous surface, but not a nitrocellulose filter.

According to a second aspect of the present invention there is provided a method for determining the presence of a specific sequence occurring within a double stranded nucleic acid in an aqueous sample comprising:

a) providing an immobilized nucleic acid probe complementary to a first sequence on either the upstream or the downstream side of said specific sequence;

b) further providing a labelled nucleic acid probe complementary to a second sequence on the other of the upstream or downstream side of said specific sequence;

c) contacting said double stranded nucleic acid in an aqueous sample

either with a restriction endonuclease which will cleave said nucleic acid strand at said specific sequence if present but not at any site between or within said first and second sequences whereby said nucleic acid strand is digested into at least two portions if said specific sequence is present

or with a restriction endonuclease which will not cleave said nucleic acid strand at said specific sequence if said specific sequence is present nor at any other site between said first or second sequence and said specific sequence or within said first and second sequences whereby said first and second sequences remain connected if said specific sequence is present;

d) denaturing said double stranded nucleic acid into single stranded nucleic acid after said contacting step;

e) reacting said single stranded nucleic acid with said immobilized nucleic acid probe and said labelled nucleic acid probe under conditions which permit hybridization to occur;

f) separating said immobilized probe from said aqueous sample; and

g) determining the presence of said specific sequence, wherein said determining step comprises detecting the presence of label in said aqueous portion following said separation step and relating same to a presence or absence of said specific sequence.

According to the third aspect of the present invention there is provided a method for determining the presence of a specific sequence occurring within a double stranded nucleic in an aqueous sample comprising:

a) providing a first immobilized nucleic acid probe complementary to a first sequence on either the upstream or the downstream side of said specific sequence;

b) further providing a second immobilized nucleic acid probe complementary to a second sequence on the other of said upstream or downstream side of said specific sequence;

c) contacting said nucleic acid with a restriction endonuclease which will cleave said nucleic acid at said specific sequence if present but not at any other site between or within said first and second sequences whereby said nucleic acid is digested into at least two portions if said specific sequence is present;

d) denaturing said nucleic acid into single stranded nucleic acid after said contacting step;

e) reacting said single stranded nucleic acid with said first and second immobilized nucleic acid probes under conditions which permit hybridization to occur; and

f) determining the presence of said specific sequence on the basis of agglutination or changes in agglutination settling rates.

According to a fourth aspect of the present invention there is provided a method for determining the presence of a specific sequence occurring within a double stranded nucleic acid in an aqueous sample comprising:

a) providing an immobilized nucleic acid probe complementary to a first sequence on either the upstream or the downstream side of the specific sequence;

b) further providing an oligonucleotide complementary to a second sequence on the other of the upstream or downstream side of the specific sequence and at least a part of the specific sequence;

c) contacting the double stranded nucleic acid in an aqueous sample

either with a restriction endonuclease which will cleave the nucleic acid strand at the specific sequence if present but not at any site between or within the first and second sequences whereby the nucleic acid strand is digested into at least two portions if the specific sequence is present

or with a restriction endonuclease which will not cleave the nucleic acid strand at the specific sequence if the specific sequence is present nor at any other site between the first or second sequence and the specific sequence or within the first and second sequences whereby the first and second sequences remain connected if the specific sequence is present;

d) denaturing the double stranded nucleic acid into single stranded nucleic acid after the contacting step;

e) reacting the single stranded nucleic acid with the immobilized nucleic acid probe and the oligonucleotide under conditions which permit hybridization to occur;

f) separating the immobilized probe from the aqueous sample; and

g) determining the presence of the specific sequence on the basis of whether or not the oligonucleotide is associated with the immobilized probe following the separation step by means of determining the melting characteristics of the immobilised nucleic acid.

In accordance with the principles and objects of the present invention there are provided methods for determining the presence of specific genetic sequences in sample nucleic acid strands useful for diagnosing the presence or absence of disease associated genetic abnormalities.

The double stranded sample DNA to be analyzed is treated with a restriction endonuclease selected to operate at the site to be analyzed. As will be apparent to those skilled in the art, the DNA will advantageously be of sufficiently high molecular weight and parity for the restriction endonuclease to be enzymatically active. The restriction endonuclease may be selected to enzymatically cleave the DNA only if the abnormality occurs, or enzymatically cleave the DNA only if the abnormality does not occur depending upon the nucleic acid sequence in those two cases and the concurrent availability of a suitable restriction endonuclease. Thus, intimate knowledge of the exact base pair sequence at the site of abnormality is required as well as the availability of a restriction endonuclease which operates at that site. Further, the restriction endonuclease will be advantageously selected so that it does not generally react anywhere else along the patient or sample nucleic acid, and specifically, elsewhere along the regions where probe hybridization will occur or regions intermediate thereof.

Activity of the restriction endonuclease at the site to be analyzed results in cleavage of the double stranded nucleic acid strand at that site thereby separating the DNA into double stranded fragments.

The double stranded DNA fragments are denatured to single stranded fragments and thereafter reacted with two nucleic acid probes. In the preferred mode, at least one of the probes is immobilized and is selected to hybridize with a base pair sequence on one side of the genetic site to be analyzed for, while the other nucleic acid probe is preferably labeled and selected to hybridize with a base pair sequence on the other side of said genetic site. Thus, the genetic site to be analyzed is intermediate the two probe hybridization regions of the sample nucleic acid. Since the sample DNA will be present in complementary single strands, the two nucleic acid probes will preferably also be provided in complementary forms to thereby permit hybridization with both single-stranded sample DNA fragments and facilitate detection.

Immobilization of the nucleic acid may be accomplished by attachment to any of a variety of solid phase means including latex particles, plastic or glass surfaces, porous surfaces such as nitrocellulose fibers or the like. Similarly the labeled probe may have attached thereto a label selected from any of the well-known detectable labels commonly employed in immunoassays. These include, for instance, enzymes, fluorescent molecules, chromophores, reflective particles such as gold particles, magnetic particles, radioisotopes and the like.

The actual physical separation may be accomplished by any suitable means such as by washing, centrifugation and the like in accordance with well-known techniques.

Analysis of the presence or absence of the label with the immobilized phase or the aqueous phase will permit determination of the presence or absence of the specific genetic sequence analyzed for.

Alternately, both probes may be immobilized such as on microparticles or latex beads and agglutination or changes in agglutination patterns related to activity of the endonuclease, in turn dependent upon the presence or absence of the base pair sequence of interest.

Detailed Description of the Invention

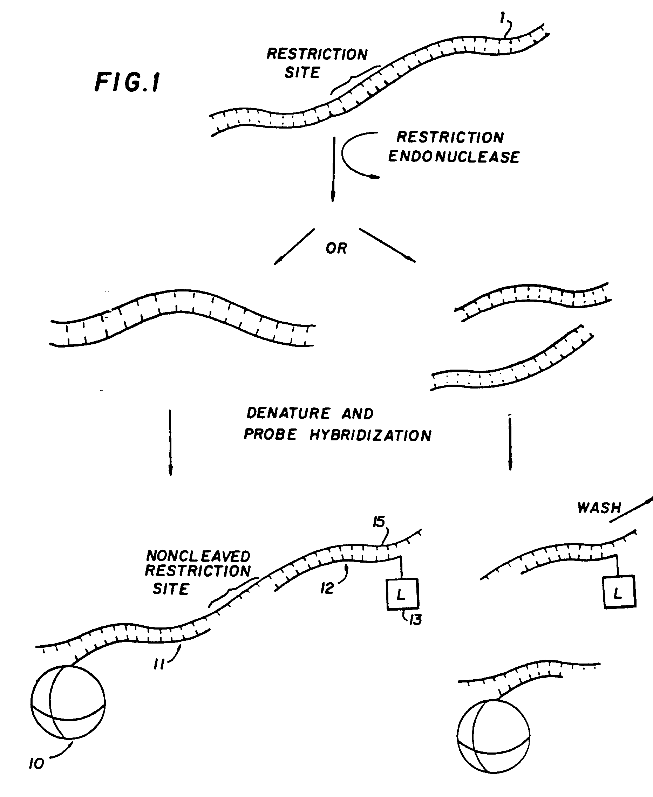

Further understanding of the operation and principles of the invention may be had by reference to the Figure which schematically portrays the preferred method of the instant invention. The double stranded sample nucleic acid strand 1 is treated with a restriction endonuclease selected to act at the site of interest. The logic relating the presence of label in the aqueous or solid phases with the presence or absence of the sequence to be analyzed for will be dependent upon the normal and abnormal base pair sequence as well as the availability of an endonuclease in either of those two circumstances. If not already apparent, this logic will become clear upon studying the Figure and the ensuing discussion.

As will be readily appreciated, activity of the restriction endonuclease will be, at least, partially dependent upon providing the sample DNA in a relatively high molecular weight and pure form. Accordingly, in some circumstances it may occasionally be necessary to perform additional preliminary steps to prepare the sample DNA to ensure adequate endonuclease activity.

The double stranded sample nucleic acid is then denatured and, treated either preferably simultaneously, or sequentially thereafter with each of two different nucleic acid probes 11 and 12. Denaturization of the double stranded DNA into single stranded DNA can be accomplished by heating an aqueous solution of DNA at elevated temperatures such as 90°C or greater or by exposing the DNA to an elevated pH such as 12 or greater. The resultant single strands are complementary and would undergo DNA renaturization if kept for a prolonged period at 65°C or under other appropriate conditions. Since there are two complementary copies, it will be advantageous to also provide complementary copies of probes 11 and 12 so that all available signal can be produced.

At least one of the nucleic acid probes 11 is preferably immobilized onto a solid phase surface 10, shown in the Figure as a latex bead or microparticle for convenience. The instant invention is not, however, so limited, and the solid phase may be any of a number of well-known formats.

These formats include, for example, latex beads of any size including the so-called macroscopic and microscopic sizes; plastic or glass surfaces such as the walls of a microtiter well, test tube and the like; porous surfaces such as the fibers in a cellulose mat employed in conventional blot hybridization techniques or any other similar structure. The only criteria is that if one of the probes is to be labeled and therefore soluble, the solid phase portion of the assay must be separable from the aqueous phase of the assay whereby the label can be detected in the aqueous phase if cleavage has occurred at the restriction site. Otherwise, if agglutination or agglutination changes are to be used to monitor endonuclease activity, then both probes will preferably be labeled with latex beads, microparticles, or the like.

Continuing with the description of the preferred mode, probe 12 is suitably labeled with label 13 which permits its ready identification and/or quantitation in either the aqueous or solid phase portions of the assay. Such probes will preferably be fluorescent, enzymatic, or isotopic in nature in order to provide the desired sensitivity, however, other labels are also contemplated and may include metallic particles, chemiluminescent or phosphorescent molecules, non-fluorescent dyes and the like. These labels can be readily detected with great sensitivity by employing appropriate instrumentation readily available commercially.

With further reference to the figure, Probes 11 and 12 will also be suitably selected in order to hybridize with the single stranded patient DNA sample on either side of the restriction site. Thus, if no endonuclease digestion occurred due to an incorrect sequence (vis-a-vis the enzyme) at the restriction site, the immobilized probe, hybridized on one side of the site, will be connected to the labeled probe, hybridized to the sample on the other side of the restriction site.

Separation of the solid and aqueous phases may then be effected by any of a variety of means which may include, for instance, a wash step such as indicated in the Figure whereby the aqueous phase containing soluble reagents is removed. Of course, other methods of separating the two phases including centrifugation etc. are equally applicable and may be selected in accordance with the investigator's expertise, available equipment, or type of insoluble phase chosen.

Following separation, either the solid phase portion or the aqueous phase portion may be examined for the presence of label. Presence of label associated with the solid phase would be indicative of little or no restriction endonuclease activity as would the absence of label in the aqueous portion. As should be readily apparent, presence of the label in the aqueous portion (or absence thereof in the solid phase portion) would therefore be indicative of restriction endonuclease activity at the cleavage site. Based on the restriction site sequence and the selected restriction endonuclease, presence or absence of the label in the solid or aqueous phases may then be related to the presence or absence of the normal or abnormal nucleotide sequence as aforesaid.

Alternately, if agglutination detection techniques are to be employed, then the separation step need not be performed. Instead the aqueous solution can be examined directly for agglutination, i.e., no enzymatic digestive activity, or still alternately, changes in agglutinate settling rates, etc., for determination of enzymatic digestive activity.

As may now be readily perceived, in order to fully benefit from the instant invention, the sequence of base pairs at the site of interest must be known or determinable. As previously indicated, many investigators have been successful in identifying disease associated genetic abnormalities and in particular, the nucleotide sequences associated therewith. Knowledge of these sequences allows the selection of an appropriate restriction endonuclease. Endonucleases are commonly available commercially from a variety of manufacturers. With the continued success in identifying disease associated nucleic sequences as well as the expected discovery of still additional restriction endonucleases, the versatility of the instant invention will continue to increase.

Similarly, the nucleic acid probes will be selected, or produced using methods now well-known, so as to hybridize with (i.e., be substantially homologous with) the single stranded nucleic acid on either side of the site of interest. One commercial supplier of labeled nucleic acid probes is Enzo Biochem of New York, New York.

The instant invention can also be utilized in a variety of oncogene studies such as assisting the identification of the bacteriophage lambda of the genomic library which carries the oncogene responsible for transformation of normal cells into tumor cells. For example, the bladder carcinoma oncogene is believed to comprise approximately 5,000 DNA base pairs. Similarly, the proto-oncogene which is the normal antecedent of the oncogene, is approximately the same size but has no transforming effect associated therewith. Since the proto-oncogene is conserved, one may conclude that there must be some associated organismic function although as yet unidentified. The protooncogene, however, differs from the oncogene in only one base pair. Such a difference could arise from a point mutation wherein the guanine base is replaced with a thymine. Alternatively, the replacement may occur by chromosomal rearrangement, gene amplification or enhanced transcription. In any case, the methods and principles of the instant invention would permit identification of the oncogene versus the proto-oncogene given the appropriate restriction endonuclease having activity at the point mutation for either the oncogene or the proto-oncogene.

Equally contemplated is the use of oligodeoxynucleotides (also known as oligonucleotides) for at least one of the nucleic acid probes. Oligonucleotides typically contain at least approximately 14 base pairs as opposed to the more conventional probe size of at least approximately 50 base pairs. With shorter probes, lower melting points will be observed and, as with the longer probes, homology can be gauged based on changes in the melting temperatures albeit at lower temperatures. The shorter Probes can permit the selection of probes which hybridize with the sample DNA over areas which encompass or overlap the restriction site. In these instances, the manner of endonuclease cleavage and probe hybridization must be carefully chosen so that if digestion at the restriction site occurs, hybridization of the probe does not take place. Thereafter, the logic relating the presence of label in the aqueous or solid phases with the presence of a particular base sequence at the restriction site proceeds as previously described. Since these conditions, even with very short probes, will be difficult to attain, the aforementioned embodiments utilizing conventional probes homologous to sequences not overlapping the restriction site are preferred.

Various alternatives to the aforementioned, including the rearrangement of steps and other similar variations, will become readily apparent to those skilled in the art, however, all such variations are to be deemed equivalents within the spirit and scope of the present invention and as defined by the following claims.

**Claims**

1. A method for determining the presence of a specific sequence occurring within a double stranded nucleic acid in an aqueous sample comprising:

a) providing an immobilized nucleic acid probe complementary to a first sequence on either the upstream or the downstream side of said specific sequence;

b) further providing a labelled nucleic acid probe complementary to a second sequence on the other of the upstream or downstream side of said specific sequence;

c) contacting said double stranded nucleic acid in an aqueous sample

either with a restriction endonuclease which will cleave said nucleic acid strand at said specific sequence if present but not at any site between or within said first and second sequences whereby said nucleic acid strand is digested into at least two portions if said specific sequence is present

or with a restriction endonuclease which will not cleave said nucleic acid strand at said specific sequence if said specific sequence is present nor at any other site between said first or second sequence and said specific sequence or within said first and second sequences whereby said first and second sequences remain connected if said specific sequence is present;

d) denaturing said double stranded nucleic acid into single stranded nucleic acid after said contacting step;

e) reacting said single stranded nucleic acid with said immobilized nucleic acid probe and said labelled nucleic acid probe under conditions which permit hybridization to occur;

f) separating said immobilized probe from said aqueous sample; and

g) determining the presence of said specific sequence on the basis of whether or not said labelled probe is associated with said immobilized probe or with said aqueous sample following said separation step;

wherein said immobilized nucleic acid probe is immobilized by attachment to microbeads, latex particles, a plastic surface, a glass surface or a porous surface, but not a nitrocellulose filter.

2. The method of claim 1, wherein the restriction endonuclease cleaves at said specific base pair sequence, in which said determining step comprises either detecting the presence or absence of label associated with said immobilized probe and relating said presence or absence to an absence or presence of said specific sequence respectively; or detecting the presence or absence of label in said aqueous portion and relating same to a presence or absence of said specific sequence respectively.

3. The method of claim 1, wherein said restriction endonuclease does not cleave at said specific sequence, in which said determining step comprises either detecting the presence or absence of label associated with said immobilized probe and relating said presence or absence to presence or absence of said specific sequence respectively; or detecting the presence or absence of label in said aqueous portion and relating same to an absence or presence of said specific sequence respectively.

4. A method for determining the presence of a specific sequence occurring within a double stranded nucleic acid in an aqueous sample comprising:

a) providing an immobilized nucleic acid probe complementary to a first sequence on either the upstream or the downstream side of said specific sequence;

b) further providing a labelled nucleic acid probe complementary to a second sequence on the other of the upstream or downstream side of said specific sequence;

c) contacting said double stranded nucleic acid in an aqueous sample

either with a restriction endonuclease which will cleave said nucleic acid strand at said specific sequence if present but not at any site between or within said first and second sequences whereby said nucleic acid strand is digested into at least two portions if said specific sequence is present

or with a restriction endonuclease which will not cleave said nucleic acid strand at said specific sequence if said specific sequence is present nor at any other site between said first or second sequence and said specific sequence or within said first and second sequences whereby said first and second sequences remain connected if said specific sequence is present;

d) denaturing said double stranded nucleic acid into single stranded nucleic acid after said contacting step;

e) reacting said single stranded nucleic acid with said immobilized nucleic acid probe and said labelled nucleic acid probe under conditions which permit hybridization to occur;

f) separating said immobilized probe from said aqueous sample; and

g) determining the presence of said specific sequence, wherein said determining step comprises detecting the presence of label in said aqueous portion following said separation step and relating same to a presence or absence of said specific sequence.

5. The method of claim 4 wherein the restriction endonuclease cleaves at said specific base pair sequence.

6. The method according to claim 4 or 5 wherein said immobilized nucleic acid probe is a immobilized by attachment to microbeads, latex particles, a plastic surface, a glass surface or a porous surface.

7. The method according to any one of the preceding claims wherein said label is radioisotope, a flurorophore, a chromophore, an enzyme, magnetic particles, light scattering particles or reflective metallic particles.

8. The method of claim 7, wherein the label is $^{32}$P, $^{131}$I, $^{125}$I, or $^{35}$S.

9. The method according to any one of the preceding claims wherein said separation step comprises washing or centrifuging.

10. A method for determining the presence of a specific sequence occurring within a double stranded nucleic in an aqueous sample comprising:

a) providing a first immobilized nucleic acid probe complementary to a first sequence on either the upstream or the downstream side of said specific sequence;

b) further providing a second immobilized nucleic acid probe complementary to a second sequence on the other of said upstream or downstream side of said specific sequence;

c) contacting said nucleic acid with a restriction endonuclease which will cleave said nucleic acid at said specific sequence if present but not at any other site between or within said first and second sequences whereby said nucleic acid is digested into at least two portions if said specific sequence is present;

d) denaturing said nucleic acid into single stranded nucleic acid after said contacting step;

e) reacting said single stranded nucleic acid with said first and second immobilized nucleic acid probes under conditions which permit hybridization to occur; and

f) determining the presence of said specific sequence on the basis of agglutination or changes in agglutination settling rates.

11. The method of claim 10 wherein said first and second nucleic acid probes are immobilized by attachment to microbeads, macrobeads, latex particles or metallic particles.

12. The method according to any one of the preceding claims wherein said specific sequence

8

is associated with a genetic abnormality.

13. The method according to any one of claims 10 to 12 wherein a decrease in agglutination settling rate is related to restriction endonuclease activity and the presence of the specific sequence.

14. A method for determining the presence of a specific sequence occurring within a double stranded nucleic acid in an aqueous sample comprising:
   a) providing an immobilized nucleic acid probe complementary to a first sequence on either the upstream or the downstream side of the specific sequence;
   b) further providing an oligonucleotide complementary to a second sequence on the other of the upstream or downstream side of the specific sequence and at least a part of the specific sequence;
   c) contacting the double stranded nucleic acid in an aqueous sample
      either with a restriction endonuclease which will cleave the nucleic acid strand at the specific sequence if present but not at any site between or within the first and second sequences whereby the nucleic acid strand is digested into at least two portions if the specific sequence is present
      or with a restriction endonuclease which will not cleave the nucleic acid strand at the specific sequence if the specific sequence is present nor at any other site between the first or second sequence and the specific sequence or within the first and second sequences whereby the first and second sequences remain connected if the specific sequence is present;
   d) denaturing the double stranded nucleic acid into single stranded nucleic acid after the contacting step;
   e) reacting the single stranded nucleic acid with the immobilized nucleic acid probe and the oligonucleotide under conditions which permit hybridization to occur;
   f) separating the immobilized probe from the aqueous sample; and
   g) determining the presence of the specific sequence on the basis of whether or not the oligonucleotide is associated with the immobilized probe following the separation step by means of determining the melting characteristics of the immobilised nucleic acid.

15. A method according to claim 14 wherein the oligonucleotide is complementary to the second sequence and all of the restriction site.

16. A method according to claim 14 or 15, wherein the immobilized nucleic acid probe is immobilized by attachment to microbeads, latex particles, a plastic surface, a glass surface or a porous surface.

17. A method according to claim 16 wherein the immobilised nucleic acid probe is immobilised to nitrocellulose paper.

18. A method according to any one of claims 14 to 17 wherein the restriction endonuclease cleaves at the specific base pair sequence, in which the determining step comprises determining the presence or absence of oligonucleotide associated with the immobilized probe and relating the presence or absence to an absence or presence of the specific sequence respectively.

19. A method according to any one of claims 14 to 18 wherein the restriction endonuclease does not cleave at the specific sequence, in which the determining step comprises determining the presence or absence of oligonucleotide associated with the immobilized probe and relating the presence or absence to presence or absence of the specific sequence respectively.

**Patentansprüche**

1. Verfahren zur Bestimmung des Vorhandenseins einer spezifischen, innerhalb einer doppelsträngigen Nucleinsäure vorkommenden Sequenz in einer wäßrigen Probe, umfassend:
   a) Bereitstellen einer immobilisierten Nucleinsäuresonde, die zu einer ersten Sequenz, die sich entweder auf der stromaufwärts oder stromabwärts liegenden Seite der spezifischen Sequenz befindet, komplementär ist;
   b) ferner Bereitstellen einer markierten Nucleinsäuresonde, die zu einer zweiten Sequenz, die sich auf der anderen stromaufwärts oder stromabwärts liegenden Seite der spezifischen Sequenz befindet, komplementär ist;
   c) Zusammenbringen der doppelsträngigen Nucleinsäure in einer wäßrigen Probe
      entweder mit einer Restriktionsendonuclease, die den Nucleinsäurestrang an der spezifischen Sequenz spalten wird, wenn die spezifische Sequenz vorhanden ist, aber nicht an einer Stelle zwischen oder innerhalb der ersten und zweiten Sequenz, wobei der Nucleinsäurestrang in mindestens zwei Teile gespalten wird, wenn die spezifische Sequenz vorhanden ist,

oder mit einer Restriktionsendonuclease, die den Nucleinsäurestrang an der spezifischen Sequenz nicht spalten wird, wenn die spezifische Sequenz vorhanden ist, und auch nicht an einer anderer Stelle zwischen der ersten oder zweiten Sequenz und der spezifischen Sequenz oder innerhalb der ersten und zweiten Sequenz, wobei die erste und zweite Sequenz verbunden bleiben, wenn die spezifische Sequenz vorhanden ist;

d) Denaturieren der doppelsträngigen Nucleinsäure in einzelsträngige Nucleinsäure nach dem Schritt des Zusammenbringens;

e) Umsetzen der einzelsträngigen Nucleinsäure mit der immobilisierten Nucleinsäuresonde und der markierten Nucleinsäuresonde unter Bedingungen, die eine Hybridisierung ermöglichen;

f) Trennen der immobilisierten Sonde von der wäßrigen Probe; und

g) Bestimmen des Vorhandenseins der spezifischen Sequenz basierend darauf, ob oder ob nicht die markierte Sonde mit der immobilisierten Sonde oder mit der wäßrigen Probe nach dem Trennschritt verbunden ist;

wobei dem die immobilisierte Nucleinsäuresonde durch Anheftung an Mikrokügelchen, Latexpartikel, einer Kunststoffoberfläche, einer Glasoberfläche oder einer porösen Oberfläche, aber nicht an einem Nitrocellulosefilter, immobilisiert ist.

2.  Verfahren nach Anspruch 1, wobei die Restriktionsendonuclease an der spezifischen Basenpaarsequenz spaltet, in dem der Bestimmungsschritt entweder den Nachweis des Vor- oder Nichtvorhandenseins der mit der immobilisierten Sonde verbundenen Markierung und den Bezug dieses Vor- oder Nichtvorhandenseins auf ein Vor- bzw. Nichtvorhandenseins der spezifischen Sequenz umfaßt, oder den Nachweis des Vor- oder Nichtvorhandenseins der Markierung in dem wäßrigen Teil und dessen Bezug auf ein Vor- bzw. Nichtvorhandenseins der spezifischen Sequenz umfaßt.

3.  Verfahren nach Anspruch 1, wobei die Restriktionsendonuclease nicht an der spezifischen Sequenz spaltet, in dem der Bestimmungsschritt entweder den Nachweis des Vor- oder Nichtvorhandenseins der mit der immobilisierten Sonde verbundenen Markierung und den Bezug des Vor- oder Nichtvorhandenseins auf ein Vor- bzw. Nichtvorhandenseins der spezifischen Sequenz umfaßt, oder den Nachweis dem Vor- oder Nichtvorhandenseins der Markierung in dem wäßrigen Teil und dessen Bezug auf ein Vor- bzw. Nichtvorhandenseins der spezifischen Sequenz umfaßt.

4.  Verfahren zur Bestimmung des Vorhandenseins einer spezifischen, innerhalb einer doppelsträngigen Nucleinsäure vorkommenden Sequenz in einer wäßrigen Probe, umfassend:

a) Bereitstellen einer immobilisierten Nucleinsäuresonde, die zu einer ersten Sequenz, die sich entweder auf der stromaufwärts oder stromabwärts liegenden Seite der spezifischen Sequenz befindet, komplementär ist;

b) ferner Bereitstellen einer markierten Nucleinsäuresonde, die zu einer zweiten Sequenz, die sich auf der anderen stromaufwärts oder stromabwärts liegenden Seite der spezifischen Sequenz befindet, komplementär ist;

c) Zusammenbringen der doppelsträngigen Nucleinsäure in einer wäßrigen Probe entweder mit einer Restriktionsendonuclease, die den Nucleinsäurestrang an der spezifischen Sequenz spalten wird, wenn die spezifische Sequenz vorhanden ist, aber nicht an einer Stelle zwischen oder innerhalb der ersten und zweiten Sequenz, wobei der Nucleinsäurestrang in mindestens zwei Teile gespalten wird, wenn die spezifische Sequenz vorhanden ist,

oder mit einer Restriktionsendonuclease, die den Nucleinsäurestrang an der spezifischen Sequenz nicht spalten wird, wenn die spezifische Sequenz vorhanden ist, und auch nicht an einer anderen Stelle zwischen der ersten oder zweiten Sequenz und der spezifischen Sequenz oder innerhalb der ersten und zweiten Sequenz, wobei die erste und zweite Sequenz verbunden bleiben, wenn die spezifische Sequenz vorhanden ist;

d) Denaturieren der doppelsträngigen Nucleinsäure in einzelsträngige Nucleinsäure nach dem Schritt des Zusammenbringens;

e) Umsetzen der einzelsträngigen Nucleinsäure mit der immobilisierten Nucleinsäuresonde und der markierten Nucleinsäuresonde unter Bedingungen, die eine Hybridisierung ermöglichen;

f) Trennen der immobilisierten Sonde von der wäßrigen Probe, und

g) Bestimmen des Vorhandenseins der spezifischen Sequenz, wobei der Bestimmungsschritt den Nachweis des Vorhandenseins der Markierung in dem wäßrigen Teil nach dem Trennungsschritt und dessen Bezug auf ein Vor- oder Nichtvorhandenseins der spezifischen Sequenz umfaßt.

5. Verfahren nach Anspruch 4, wobei die Restriktionsendonuclease an der spezifischen Basenpaarsequenz spaltet.

6. Verfahren nach Anspruch 4 oder 5, wobei die immobilisierte Nucleinsäuresonde durch Anheftung an Mikrokügelchen, Latexpartikel, eine Kunststoffoberfläche, eine Glasoberfläche oder eine poröse Oberfläche immobilisiert ist.

7. Verfahren nach einem der vorangehenden Ansprüche, wobei die Markierung ein Radioisotop, ein Fluorophor, ein Chromophor, ein Enzym, magnetische Partikel, lichtbrechende Partikel oder reflektierende metallische Partikel sind.

8. Verfahren nach Anspruch 7, wobei die Markierung $^{32}$P, $^{131}$J, $^{125}$J oder $^{35}$S ist.

9. Verfahren nach einem der vorangehenden Ansprüche, wobei der Trennschritt Waschen oder Zentrifugieren umfaßt.

10. Verfahren zur Bestimmung des Vorhandenseins einer spezifischen, innerhalb einer doppelsträngigen Nucleinsäure vorkommenden Sequenz in einer wäßrigen Probe, umfassend:
    a) Bereitstellen einer immobilisierten Nucleinsäuresonde, die zu einer ersten Sequenz, die sich entweder auf der stromaufwärts oder stromabwärts liegenden Seite der spezifischen Sequenz befindet, komplementär ist;
    b) ferner Bereitstellen einer markierten Nucleinsäuresonde, die zu einer zweiten Sequenz, die sich auf der anderen stromaufwärts oder stromabwärts liegenden Seite der spezifischen Sequenz befindet, komplementär ist;
    c) Zusammenbringen der Nucleinsäure mit einer Restriktionsendonuclease, die die Nucleinsäure an der spezifischen Sequenz spalten wird, wenn die spezifische Sequenz vorhanden ist, aber nicht an einer anderen Stelle zwischen oder innerhalb der ersten und zweiten Sequenz, wobei die Nucleinsäure in mindestens zwei Teile gespalten wird, wenn die spezifische Sequenz vorhanden ist;
    d) Denaturieren der Nucleinsäure in einzelsträngige Nucleinsäure nach dem Schritt des Zusammenbringens;
    e) Umsetzen der einzelsträngigen Nucleinsäure mit der ersten und zweiten immobilisierten Nucleinsäuresonde unter Bedingungen, die eine Hybridisierung ermöglichen; und

f) Bestimmen dem Vorhandenseins der spezifischen Sequenz auf der Grundlage der Agglutination oder Änderungen in der Agglutinations-Absetzrate.

11. Verfahren nach Anspruch 10, wobei die erste und zweite Nucleinsäuresonde durch Anheften an Mikrokügelchen, Makrokügelchen, Latexpartikel oder metallische Partikel immobilisiert sind.

12. Verfahren nach einen der vorangehenden Ansprüche, wobei die spezifische Sequenz mit einer genetischen Abnormalität verbunden ist.

13. Verfahren nach einem der Ansprüche 10 bis 12, wobei eine Verringerung in der Agglutinations-Absetzrate in Bezug gesetzt wird mit der Restriktionsendonucleaseaktivität und dem Vorhandensein der spezifischen Sequenz.

14. Verfahren zur Bestimmung des Vorhandenseins einer spezifischen, innerhalb einer doppelsträngigen Nucleinsäure vorkommenden Sequenz in einer wäßrigen Probe, umfassend:
    a) Bereitstellen einer immobilisierten Nucleinsäuresonde, die zu einer ersten Sequenz, die sich entweder auf der stromaufwärts oder stromabwärts liegenden Seite der spezifischen Sequenz befindet, komplementär ist;
    b) ferner Bereitstellen eines markierten Oligonucleotid, das zu einer zweiten Sequenz, die sich auf der anderen stromaufwärts oder stromabwärts liegenden Seite der spezifischen Sequenz befindet, komplementär ist;
    c) Zusammenbringen der doppelsträngigen Nucleinsäure in einer wäßrigen Probe entweder mit einer Restriktionsendonuclease, die den Nucleinsäurestrang an der spezifischen Sequenz spalten wird, wenn die spezifische Sequenz vorhanden ist, aber nicht an einer Stelle zwischen oder innerhalb der ersten und zweiten Sequenz, wobei der Nucleinsäurestrang in mindestens zwei Teile gespalten wird, wenn die spezifische Sequenz vorhanden ist, oder mit einer Restriktionsendonuclease, die den Nucleinsäurestrang an der spezifischen Sequenz nicht spalten wird, wenn die spezifische Sequenz vorhanden ist, und auch nicht an einer anderen Stelle zwischen der ersten oder zweiten Sequenz und der spezifischen Sequenz oder innerhalb der ersten und zweiten Sequenz, wobei die erste und zweite Sequenz verbunden bleiben, wenn die spezifische Sequenz vorhanden ist;

d) Denaturieren der doppelsträngigen Nucleinsäure in einzelsträngige Nucleinsäure nach dem Schritt des Zusammenbringens;

e) Umsetzen der einzelsträngigen Nucleinsäure mit der immobilisierten Nucleinsäure und dem Oligonucleotid unter Bedingungen, die eine Hybridisierung ermöglichen;

f) Trennen der immobilisierten Sonde Von der wäßrigen Probe; und

g) Bestimmen des Vorhandenseins der spezifischen Sequenz basierend darauf, ob oder ob nicht das Oligonucleotid mit der immobilisierten Sonde nach dem Trennschritt verbunden ist, indem die Schmelzeigenschaften der immobilisierten Nucleinsäure bestimmt werden.

15. Verfahren nach Anspruch 14, wobei das Oligonucleotid zu der zweiten Sequenz und zur gesamten Restriktionsstelle komplementär ist.

16. Verfahren nach Anspruch 14 oder 15, wobei die immobilisierte Nucleinsäuresonde durch Anheftung an Mikrokügelchen, Latexpartikel, eine Kunststoffoberfläche, eine Glasoberfläche oder eine poröse Oberfläche immobilisiert ist.

17. Verfahren nach Anspruch 16, wobei die immobilisierte Nucleinsäuresonde auf Nitrocellulosepapier immobilisiert ist.

18. Verfahren nach einem der Ansprüche 14 bis 17, wobei die Restriktionsendonuclease an der spezifischen Basenpaarsequenz spaltet, in dem der Bestimmungsschritt den Nachweis des Vor- oder Nichtvorhandenseins des mit der immobilisierten Sonde verbundenen Oligonucleotids und den Bezog des Vor- oder Nichtvorhandenseins auf ein Vor- bzw. Nichtvorhandensein der spezifischen Sequenz umfaßt.

19. Verfahren nach einem der Ansprüche 14 bis 18, wobei die Restriktionsendonuclease an der spezifischen Sequenz nicht spaltet, in dem der Bestimmungsschritt den Nachweis des Vor- oder Nichtvorhandenseins des mit der immobilisierten Sonde verbundenen Oligonucleotids und den Bezug des Vor- oder Nichtvorhandenseins auf ein Vor- bzw. Nichtvorhandensein der spezifischen Sequenz umfaßt.

**Revendications**

1. Procédé de détermination de la présence d'une séquence spécifique dans un acide nucléique bicaténaire au sein d'un échantillon aqueux, selon lequel :

a) on prépare une sonde d'acide nucléique immobilisée, complémentaire d'une première séquence du côté amont ou aval de ladite séquence spécifique ;

b) on prépare en outre une sonde d'acide nucléique marquée, complémentaire d'une deuxième séquence de l'autre côté amont ou aval de ladite séquence spécifique ;

c) on met en contact cet acide nucléique bicaténaire, dans un échantillon aqueux, avec une endonucléase de restriction qui coupe ce brin d'acide nucléique au niveau de ladite séquence spécifique, si elle est présente, mais pas au niveau d'un site quelconque entre ou à l'intérieur des première et deuxième séquences, de sorte que le brin d'acide nucléique est digéré en au moins deux parties, si ladite séquence spécifique est présente ;

ou avec une endonucléase de restriction qui ne coupe pas ledit brin d'acide nucléique au niveau de la séquence spécifique, si cette séquence spécifique est présente, ni au niveau d'un autre site quelconque entre la première ou la deuxième séquence et la séquence spécifique, ou dans les première et deuxième séquences, de sorte que les première et deuxième séquences restent liées si ladite séquence spécifique est présente ;

d) on dénature l'acide nucléique bicaténaire en un acide nucléique monocaténaire après cette opération de mise en contact ;

e) on fait réagir l'acide nucléique monocaténaire avec ladite sonde d'acide nucléique immobilisée et la sonde d'acide nucléique marquée, dans des conditions permettant à l'hybridation d'avoir lieu ;

f) on sépare la sonde immobilisée de l'échantillon aqueux ; et

g) on détermine la présence de la séquence spécifique, d'après le fait que ladite sonde marquée est associée ou non avec la sonde immobilisée ou avec l'échantillon aqueux après ladite opération de séparation ;

la sonde d'acide nucléique immobilisée étant immobilisée par liaison sur des microperles, des particules de latex, une surface de matière plastique, une surface de verre ou une surface poreuse, mais pas à un filtre de nitrocellulose.

2. Procédé selon la revendication 1, dans lequel l'endonucléase de restriction coupe au niveau de ladite séquence de paires de bases spécifique, l'opération de détermination comprenant la détection de la présence ou de l'absence d'un marqueur associé à la sonde immobilisée, et la corrélation de cette présence ou absence respectivement avec une absence ou une présence de ladite séquence spécifique ; ou la détection de la présence ou de l'absence d'un marqueur dans la fraction aqueuse et sa corrélation respectivement avec une présence ou une absence de ladite séquence spécifique.

3. Procédé selon la revendication 1, dans lequel l'endonucléase de restriction ne coupe pas au niveau de ladite séquence spécifique, l'opération de détermination comprenant la détection de la présence ou de l'absence d'un marqueur associé à la sonde immobilisée et la corrélation de cette présence ou absence respectivement avec la présence ou l'absence de ladite séquence spécifique ; ou la détection de la présence ou de l'absence d'un marqueur dans ladite fraction aqueuse et sa corrélation respectivement avec une absence ou une présence de ladite séquence spécifique.

4. Procédé de détermination de la présence d'une séquence spécifique dans un acide nucléique bicaténaire au sein d'un échantillon aqueux, selon lequel :

a) on prépare une sonde d'acide nucléique immobilisée, complémentaire d'une première séquence du côté amont ou aval de ladite séquence spécifique ;

b) on prépare en outre une séquence d'acide nucléique marquée, complémentaire d'une deuxième séquence de l'autre côté amont ou aval de ladite séquence spécifique ;

c) on met en contact l'acide nucléique bicaténaire dans un échantillon aqueux, avec une endonucléase de restriction qui coupe ledit brin d'acide nucléique au niveau de ladite séquence spécifique si elle est présente, mais pas au niveau d'un site quelconque entre ou à l'intérieur des première et deuxième séquences, de sorte que le brin d'acide nucléique est digéré en au moins deux parties si la séquence spécifique est présente ;

ou avec une endonucléase de restriction qui ne coupe pas le brin d'acide nucléique au niveau de ladite séquence spécifique, si cette séquence spécifique est présente, ni au niveau d'un autre site quelconque entre la première ou la deuxième séquence et la séquence spécifique ou dans les première et deuxième séquences, de sorte que les première et deuxième séquences restent liées si ladite séquence spécifique est présente ;

d) on dénature l'acide nucléique bicaténaire en un acide nucléique monocaténaire après l'opération de mise en contact ;

e) on fait réagir l'acide nucléique monocaténaire avec la sonde d'acide nucléique immobilisée et la sonde d'acide nucléique marquée, dans des conditions permettant à l'hybridation d'avoir lieu ;

f) on sépare la sonde immobilisée de l'échantillon aqueux ; et

g) on détermine la présence de la séquence spécifique, l'opération de détermination comprenant la détection de la présence du marqueur dans la fraction aqueuse après l'opération de séparation, et la corrélation de celle-ci avec la présence ou à l'absence de la séquence spécifique.

5. Procédé selon la revendication 4, dans lequel l'endonucléase de restriction coupe au niveau de ladite séquence de paires de bases spécifique.

6. Procédé selon la revendication 4 ou 5, dans lequel la sonde d'acide nucléique immobilisée, est immobilisée par liaison avec des microperles, des particules de latex, une surface de matière plastique, une surface de verre ou une surface poreuse.

7. Procédé selon l'une quelconque des revendications précédentes, dans lequel le marqueur est un radioisotope, un fluorophore, un chromophore, une enzyme, des particules magnétiques, des particules dispersant la lumière ou des particules métalliques réfléchissantes.

8. Procédé selon la revendication 7, dans lequel le marqueur est $^{32}$P, $^{131}$I, $^{125}$I, ou $^{35}$S.

9. Procédé selon l'une quelconque des revendications précédentes, dans lequel l'opération de séparation comprend un lavage ou une centrifugation.

10. Procédé de détermination de la présence d'une séquence spécifique dans un acide nucléique bicaténaire au sein d'un échantillon aqueux, selon lequel :

a) on prépare une première sonde d'acide nucléique immobilisée, complémentaire d'une première séquence du côté amont ou aval de ladite séquence spécifique ;

b) on prépare en outre une deuxième sonde d'acide nucléique immobilisée, complémentaire d'une deuxième séquence de l'autre côté amont ou aval de la séquence spécifique ;

c) on met en contact l'acide nucléique avec une endonucléase de restriction qui coupe cet acide nucléique au niveau de ladite séquence spécifique si elle est présente, mais pas au niveau d'un autre site quelconque entre ou à l'intérieur des première et deuxième séquences, de sorte que l'acide nucléique est digéré en au moins deux fragments si ladite séquence spécifique est présente ;

d) on dénature l'acide nucléique en un acide nucléique monocaténaire après l'opération de mise en contact ;

e) on fait réagir l'acide nucléique monocaténaire avec les première et deuxième sondes d'acide nucléique immobilisées, dans des conditions permettant à l'hybridation d'avoir lieu ; et

f) on détermine la présence de la séquence spécifique, d'après l'agglutination ou les variations des vitesses de décantation par agglutination.

11. Procédé selon la revendication 10, dans lequel les première et deuxième sondes d'acide nucléique sont immobilisées par liaison avec des micro-perles, des macro-perles, des particules de latex ou des particules métalliques.

12. Procédé selon l'une quelconque des revendications précédentes, dans lequel la séquence spécifique est associée à une anomalie génétique.

13. Procédé selon l'une quelconque des revendications 10 à 12, dans lequel une diminution de vitesse de décantation par agglutination est associée à une activité d'endonucléase de restriction et à la présence de la séquence spécifique.

14. Procédé de détermination de la présence d'une séquence spécifique dans un acide nucléique bicaténaire au sein d'un échantillon aqueux, selon lequel :

a) on prépare une sonde d'acide nucléique immobilisée, complémentaire d'une première séquence du côté amont ou aval de la séquence spécifique ;

b) on prépare en outre un oligonucléotide complémentaire d'une deuxième séquence de l'autre côté amont ou aval de la séquence spécifique et d'au moins une partie de la séquence spécifique ;

c) on met en contact l'acide nucléique bicaténaire dans un échantillon aqueux, avec une endonucléase de restriction qui coupe le brin d'acide nucléique au niveau de la séquence spécifique si elle est présente, mais pas au niveau d'un site quelconque entre ou à l'intérieur des première et deuxième séquences, de sorte que le brin d'acide nucléique est digéré en au moins deux fragments si la séquence spécifique est présente ;

ou avec une endonucléase de restriction qui ne coupe pas le brin d'acide nucléique au niveau de la séquence spécifique, si la séquence spécifique est présente, ni au niveau d'un autre site quelconque entre la première ou la deuxième séquence et la séquence spécifique, ou à l'intérieur des première et deuxième séquences, de sorte que les première et deuxième séquences restent liées si la séquence spécifique est présente ;

d) on dénature l'acide nucléique bicaténaire en un acide nucléique monocaténaire après l'opération de mise en contact ;

e) on fait réagir l'acide nucléique monocaténaire avec la sonde d'acide nucléique immobilisée et l'oligonucléotide, dans des conditions permettant à l'hybridation d'avoir lieu ;

f) on sépare la sonde immobilisée de l'échantillon aqueux ; et

g) on détermine la présence de la séquence spécifique, d'après le fait que l'oligonucléotide est associé ou non avec la sonde immobilisée après l'opération de séparation, par détermination des caractéristiques de fusion de l'acide nucléique immobilisé.

15. Procédé selon la revendication 14, dans lequel l'oligonucléotide est complémentaire de la deuxième séquence et de la totalité du site de restriction.

16. Procédé selon la revendication 14 ou 15, dans lequel la sonde d'acide nucléique immobilisée, est immobilisée par liaison à des micro-perles, des particules de latex, une surface de matière plastique, une surface de verre ou une surface poreuse.

17. Procédé selon la revendication 16, dans lequel la sonde d'acide nucléique immobilisée, est immobilisée sur du papier de nitrocellulose.

18. Procédé selon l'une quelconque des revendications 14 à 17, dans lequel l'endonucléase de restriction coupe au niveau de la séquence de paires de bases spécifique, l'opération de détermination comprenant la détermination de la présence ou de l'absence d'un oligonucléotide associé à la sonde immobilisée, et la corrélation de la présence ou de l'absence respectivement avec une absence ou une présence de la séquence spécifique.

19. Procédé selon l'une quelconque des revendications 14 à 18, dans lequel l'endonucléase de restriction ne coupe pas au niveau de la séquence spécifique, l'opération de détermination comprenant la détermination de la présence ou de l'absence d'un oligonucléotide associé à la sonde immobilisée, et la corrélation de la présence ou de l'absence respectivement avec une présence ou une absence de la séquence spécifique.

FIG.1

RESTRICTION SITE

RESTRICTION ENDONUCLEASE

OR

DENATURE AND PROBE HYBRIDIZATION

WASH

NONCLEAVED RESTRICTION SITE